# EUROPÄISCHE PATENTSCHRIFT

(11) **EP 1 785 086 B1**
(45) Veröffentlichungstag und Bekanntmachung des Hinweises auf die Patenterteilung: **30.05.2018**
(21) Anmeldenummer: 06021578.7
(22) Anmeldetag: 14.10.2006
(51) Int. Cl.: A61B 5/00

(54) **Implantierbares Elektrodensystem und Vorrichtung zum Messen einer Analytkonzentration in einem menschlichen oder tierischen Körper**
Implantable electrode system and device for measuring the concentration of an analyte in a human or animal body
Système implantable d'électrodes et dispositif de mesure de la concentration d'un analyte dans un corps humain ou animal

(30) Priorität: 12.11.2005 EP 05024760
(43) Veröffentlichungstag der Anmeldung: 16.05.2007
(73) Patentinhaber: Roche Diabetes Care GmbH, 68305 Mannheim (DE); F. Hoffmann-La Roche AG, 4070 Basel (CH)
(72) Erfinder: Staib, Arnulf, 64646 Heppenheim (DE); Gillen, Ralph, 26871 Papenburg (DE)
(74) Vertreter: Mommer, Niels

(56) Entgegenhaltungen:
- US-A- 4 655 880
- US-A1- 2005 051 427
- US-A1- 2005 059 871
- US-A1- 2005 103 625
- US-A1- 2005 221 276
- US-B1- 6 175 752
- QU Y ET AL: "Simultaneous determination of total and extracellular concentrations of the amino acid neurotransmitters in cat visual cortex by microbore liquid chromatography and electrochemical detection" JOURNAL OF CHROMATOGRAPHY A, ELSEVIER, AMSTERDAM, NL, Bd. 798, Nr. 1-2, 6. März 1998 (1998-03-06), Seiten 19-26, XP004112336 ISSN: 0021-9673

## Beschreibung

Die Erfindung betrifft ein implantierbares Elektrodensystem, eine Vorrichtung mit einem derartigem Elektrodensystem und ein entsprechendes Verfahren zum Messen einer Analytkonzentration in einem menschlichen oder tierischem Körper. Ein derartiges Elektrodensystem ist beispielsweise aus der US 6175752 B1 bekannt.

Aus der US 2005/0103625 A1, der US 2004/0111017 A1 sowie aus der WO 2005/032362 A2 ist jeweils ein implantierbares Elektrodensystem zum Messen der Glukosekonzentration bekannt, das aus drei Elektroden besteht, nämlich einer Messelektrode, einer Gegenelektrode und einer Referenzelektrode.

Aus der US 4 655 880 ist ein Elektrodensystem mit mehreren Messelektroden und einer gemeinsamen Gegenelektrode bekannt.

Die US 2005/0051427 A1 beschreibt ein implantierbares Elektrodensystem, das hergestellt wird, indem zunächst ein Stapel aus Mess- und Gegenelektrodenmaterial sowie Isolationsschichten gebildet wird. Dieser Stapel wird anschließend aufgerollt und längs oder quer in Streifen geschnitten. Die Oberfläche der Streifen kann zusätzlich bearbeitet werden, um ebene, gerundete oder gestufte Oberflächen zu erzeugen. Bei einer gerundeten Oberfläche haben einzelnen Bereiche aus Elektrodenmaterial dann unterschiedlich große Flächen. Bei einer Messung liefern die einzelnen Messelektrodenbereiche eine Signalstärke, die im Wesentlichen der einer durchgehenden Elektrodenschicht entspricht.

Aus der WO 2004/041082 A1 sind ein Verfahren und eine Vorrichtung zum Messen der Analytkonzentration in Körperflüssigkeitsproben ex-vivo bekannt. Dabei werden photometrische oder elektrochemische Testelemente verwendet, deren Messsensitivität an unterschiedliche Konzentrationsbereiche des Analyten angepasst ist.

Aus der JP 2003-190122 A ist ein Sensor bekannt, bei dem zwei kegelförmige Elektroden auf einer Leiterplatte angebracht sind. Für eine Messung wird die Leiterplatte gegen die Haut eines Patienten gepresst, so dass die beiden kegelförmigen Elektroden durch die Haut gestochen werden.

Aus der US 2005/0221276 A1 ist ein photometrisches Sensorsystem mit mehreren Sensoren bekannt, die unterschiedliche Sensitivitäten haben und zur Messung in unterschiedlichen Konzentrationsbereichen vorgesehen sind. Die einzelnen Sensoren weisen jeweils eine enzymhaltige Membran auf, die für den Analyten beispielsweise Glucose durchlässig ist. In der Membran wird der Analyt enzymatisch in eine Säure und Wasserstoffperoxid umgewandelt. Je höher die Analytkonzentration ist, desto stärker ändert sich der pH-Wert. Die Änderung des pH-Werts wird mit einem in die Membran eingelagerten Indikator erfasst. Durch eine Farbänderung des Indikators wird somit die Analytkonzentration erfasst. In einer alternativen Ausführungsform wird die Präambel des Anspruchs 1 offenbart. Implantierbare Elektrodensysteme ermöglichen Messungen von physiologisch bedeutsamen Analyten wie beispielsweise Laktat oder Glukose in dem Körper eines Patienten. Derartige Messungen in vivo haben gegenüber üblichen Vorgehensweisen, bei denen eine Probe einer Körperflüssigkeit entnommen und außerhalb des Körpers analysiert wird, eine Reihe wichtiger Vorteile, insbesondere die Möglichkeit einer automatischen und kontinuierlichen Erfassung von Meßwerten.

Bekannte Implantate haben jedoch den Nachteil, daß sich mit ihnen Analytkonzentrationen nur mit einer geringeren Genauigkeit und Zuverlässigkeit bestimmen lassen, als es mit einer herkömmlichen ex-vivo Analyse möglich ist.

Zur Erhöhung der Meßgenauigkeit wird in der US 2005/0059871 A1 vorgeschlagen, die interessierende Analytkonzentration mit mehreren Elektroden gleichzeitig zu messen und die so erhaltenen Meßsignale durch Mittelwertbildung auszuwerten. Als zusätzliche Maßnahme wird empfohlen, ergänzend mit weiteren Sensoren andere Analytkonzentrationen oder physiologische Parameter zu bestimmen und anhand der ermittelten Konzentration von verschiedenen Analyten eine Plausibilitätsprüfung der einzelnen Resultate durchzuführen.

Trotz der auf diese Weise erzielten Verbesserungen ist es mit bekannten Elektrodensystemen nicht möglich, die Glukosekonzentration in dem Körper eines Diabetikers mit ausreichender Genauigkeit zu messen, um mit hinreichender Zuverlässigkeit vor sehr tiefen oder sehr hohen Glukosekonzentrationswerten zu warnen, die Gegenmaßnahmen des Patienten erfordern.

Aufgabe der Erfindung ist es deshalb, einen Weg aufzuzeigen, wie Analytkonzentrationen mit größerer Genauigkeit in einem menschlichen oder tierischen Körper gemessen werden kann.

Diese Aufgabe wird gelöst durch ein implantierbares Elektrodensystem mit den im Anspruch 1 angegebenen Merkmalen.

Die Aufgabe wird ferner gelöst durch eine Vorrichtung zum Messen einer Analytkonzentration in einem menschlichen oder tierischen Körper, umfassend ein derartiges Elektrodensystem, eine an das Elektrodensystem angeschlossene Auswerteeinheit zum Auswerten von Meßsignalen der ersten und der zweiten Meßelektrode, und einen Speicher, in dem mindestens ein die Meßsensitivität der ersten Meßelektrode charakterisierender erster Sensitivitätsparameter und mindestens ein die Meßsensitivität der zweiten Meßelektrode charakterisierender zweiter Sensitivitätsparameter gespeichert sind, wobei die Auswerteeinheit derart ausgebildet ist, daß durch Auswertung mindestens eines Meßsignals einer der beiden Meßelektroden feststellbar ist, in welchem Konzentrationsbereich die Analytkonzentration liegt, und falls die Analytkonzentration in dem ersten Konzentrationsbereich liegt, das Meßsignal der ersten Meßelektrode mittels des ersten Sensitivitätsparameters zum Bestimmen der Analytkonzentration ausgewertet wird, und falls die Analytkonzentration in dem zweiten Konzentrationsbereich liegt, das Meßsignal der zweiten Meßelektrode mittels des zweiten Sensitivitätsparameters zum Bestimmen der Analytkonzentration ausgewertet wird.

Bei einer derartigen Vorrichtung können die Konzentrationsbereiche der Meßelektroden überlappen. Es ist sogar möglich, daß der Konzentrationsbereich einer Meßelektrode als kleiner Teilbereich vollständig in dem wesentlich größeren Konzentrationsbereich einer anderen Meßelektrode enthalten ist. Es ist deshalb möglich, daß beispielsweise das Meßsignal der ersten Meßelektrode stets ausgewertet wird und das Meßsignal der zweiten Meßelektrode nur dann herangezogen wird, wenn die Auswertung des ersten Meßsignals ergibt, daß die Analytkonzentration in dem Konzentrationsbereich der zweiten Meßelektrode liegt.

Im Rahmen der Erfindung wurde erkannt, daß sich im menschlichen oder tierischen Körper stark schwankende Analytkonzentrationen nicht ohne Einschränkung der Meßgenauigkeit mit einer einzigen Meßelektrode bestimmen lassen. Je größer nämlich der Meßbereich einer Meßelektrode gewählt wird, desto geringer ist typischerweise die Meßsensitivität bei niedrigen Konzentrationen. Anstatt bei der Konzeption einer Meßelektrode einen optimalen Kompromiß zwischen einem möglichst großen Meßbereich und einer möglichst hohen Meßsensitivität anzustreben, wird erfindungsgemäß ein großer Meßbereich durch die Verwendung von mehreren Meßelektroden erzielt, die unterschiedliche Meßbereiche abdecken und entsprechend unterschiedliche Meßsensitivitäten haben. Indem die Meßsensitivität der ersten Meßelektrode für einen ersten Konzentrationsbereich optimiert ist und die Meßsensitivität der zweiten Meßelektrode für einen zweiten Konzentrationsbereich optimiert ist, kann ohne Beschränkung der Meßgenauigkeit ein größerer Meßbereich realisiert werden.

Unabhängig davon, ob die Analytkonzentration in der Umgebung der Meßelektroden in Bezug auf einen physiologischen Normal- oder Durchschnittswert relativ hoch oder niedrig ist, kann deshalb mit einem erfindungsgemäßen Elektrodensystem die Analytkonzentration stets mit einer Meßelektrode gemessen werden, die eine für den betreffenden Konzentrationsbereich günstige Meßsensitivität hat. Auf diese Weise kann für beliebig große Meßbereiche eine deutliche Verbesserung der Meßgenauigkeit erzielt werden.

Da mit einem erfindungsgemäßen Elektrodensystem ein wesentlich verbesserte Meßgenauigkeit erreicht werden kann, ist eine statistische Auswertung von Meßsignalen verschiedener Meßelektrode, wie sie im Stand der Technik praktiziert wird, nicht erforderlich, so daß erfindungsgemäß mit einfacheren Mitteln eine höhere Meßgenauigkeit erreicht werden kann. Selbstverständlich ist jedoch möglich in einem Elektrodensystem mehrere erste Meßelektroden für einen oberen Konzentrationsbereich und mehrere zweite Meßelektroden für einen unteren Konzentrationsbereich einzusetzen, um die Störanfälligkeit des Systems zu senken oder durch eine statistische Auswertung die Meßgenauigkeit weiter zu verbessern.

Falls in einem Elektrodensystem mehrere erste oder mehrere zweite Meßelektroden eingesetzt werden, erfordert eine statistische Auswertung zur Verbesserung der Meßgenauigkeit eine getrennte Ansteuerung der einzelnen Meßelektroden. Bei einem erfindungsgemäßen Elektrodensystem können mehrere gleiche Meßelektroden aber auch in Parallelschaltung mit einer gemeinsamen Ansteuerung angeordnet werden, so daß das Stromsignal erhöht und das Signal-Rauschverhältnis auch ohne eine statistische Auswertung verbessert werden kann. Dies ist insbesondere dann vorteilhaft, wenn die betreffenden Meßelektroden an der unteren Grenze des für sie vorgesehenen Meßbereichs messen, da das Signal-Rauschverhältnis generell um so ungünstiger ist, je kleiner die zu messende Analytkonzentration ist.

Falls aus Gründen des einfachen Aufbaus darauf verzichtet wird, mehrere gleiche Meßelektroden zu verwenden, wird bevorzugt die Fläche der Meßelektrode mit der geringeren Meßsensitivität größer, bevorzugt mindestens 50% größer, als die Fläche der Meßelektrode mit der (nächst) höheren Meßsensitivität gewählt. Auf diese Weise kann auch bei einer geringeren Meßsensitivität ein erhöhtes Stromsignal erhalten und somit das Signal-Rauschverhältnis verbessert werden.

Die Erfindung wird nachfolgend anhand von in den Figuren dargestellten Ausführungsbeispielen näher erläutert. Die darin dargestellten Besonderheiten können einzeln oder in Kombination verwendet werden, um bevorzugte Ausgestaltungen zu schaffen. Gleiche oder einander entsprechende Bauteile sind mit übereinstimmenden Bezugszeichen gekennzeichnet. Es zeigen:
- Fig. 1: ein Ablaufschema für die Ermittlung einer Analytkonzentration mit einem erfindungsgemäßen Elektrodensystem;
- Fig. 2: ein Ausführungsbeispiel eines erfindungsgemäßen Elektrodensystems;
- Fig. 3: ein weiteres Ausführungsbeispiel eines erfindungsgemäßen Elektrodensystems;
- Fig. 4: das in Figur 3 dargestellte Elektrodensystem in einer Querschnittsdarstellung;
- Fig. 5: ein Beispiel einer Kennlinie einer ersten Meßelektrode eines erfindungsgemäßen Elektrodensystems;
- Fig. 6: ein Beispiel einer Kennlinie einer zweiten Meßelektrode eines erfindungsgemäßen Elektrodensystems; und
- Fig. 7: eine schematische Darstellung einer erfindungsgemäßen Vorrichtung zum Messen einer Analytkonzentration mit einem erfindungsgemäßen Elektrodensystem.

Glukose ist ein wichtiges Beispiel für einen Analyten, dessen Konzentration im Blut und in anderen Körperflüssigkeiten eines Patienten im Tagesverlauf starken Schwankungen zwischen 40 mg/dl und 450 mg/dl unterliegen kann. Im folgendem wird deshalb ohne Beschränkung der Allgemeinheit die Ermittlung von Analytkonzentrationen am Beispiel von Messungen der Glucosekonzentration in Blut oder interstitieller Flüssigkeit erläutert.

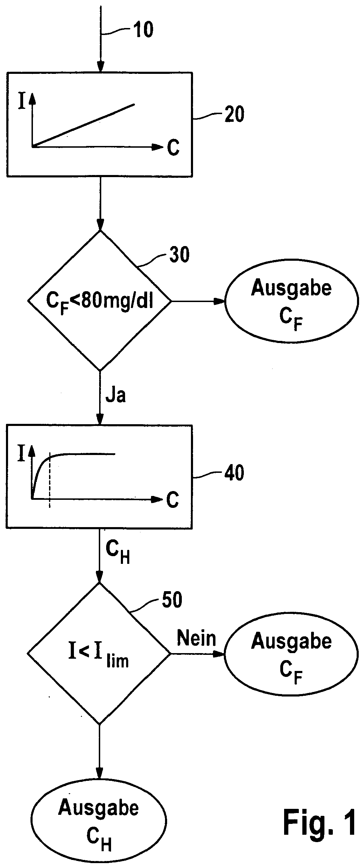

In Figur 1 ist ein Ablaufschema für die Ermittlung der Glukosekonzentration mit einem implantierbaren Elektrodensystem dargestellt, das zwei galvanisch getrennte Meßelektroden mit unterschiedlicher Sensitivität umfaßt. Eine erste Meßelektrode hat eine erste Meßsensitivität, die für einen ersten Konzentrationsbereich des Analyten von 80 mg/dl bis 500 mg/dl optimiert ist. Eine zweite Meßelektrode hat eine zweite Meßsensitivität, die für einen zweiten Konzentrationsbereich des Analyten von 20 mg/dl bis 80 mg/dl optimiert ist. Der erste Konzentrationsbereich ist also von dem zweiten Kohzentrationsbereich verschieden. Bevorzugt unterscheiden sich die Obergrenzen der Konzentrationsbereiche mindestens um einen Faktor zwei.

Die Meßelektroden liefern jeweils ein Meßsignal in Form eines elektrischen Stroms, dessen Stärke von der Analytkonzentration in der Umgebung der Meßelektroden abhängt. Für jede Meßelektrode beschreibt eine Kennlinie, die durch ein Sensitivitätsparameter charakterisiert sein kann, den Zusammenhang zwischen dem Strom I als Meßsignal und der dazugehörenden Analytkonzentration C.

Zur Bestimmung der Analytkonzentration wird in einem ersten Arbeitsschritt 10 das Meßsignal der ersten Meßelektrode mittels einer Kennlinie 20 ausgewertet und ein Wert C_{F} für die Analytkonzentration bestimmt. In einem anschließenden Schritt 30 wird geprüft, ob der anhand des Meßsignals der ersten Meßelektrode ermittelte Wert C_{F} in dem Konzentrationsbereich liegt, für den die Meßsensitivität der ersten Meßelektrode optimiert ist. Bei dem dargestellten Ausführungsbeispiel ist die erste Meßelektrode für Analytkonzentrationen von mehr als 80 mg/dl optimiert. Wird festgestellt, daß der ermittelte Wert C_{F} in dem ersten Konzentrationsbereich liegt, d. h. 80 mg/dl übersteigt, so wird der Wert C_{F} als Ergebnis ausgegeben.

Beträgt der anhand der ersten Meßelektrode ermittelte Konzentrationswert C_{F} weniger als 80 mg/dl, wird anschließend das Meßsignal der zweiten Meßelektrode anhand der Kennlinie 40 der zweiten Meßelektrode ausgewertet.

Bevorzugt hat die Kennlinie 40 der zweiten Meßelektrode bei kleinen Konzentrationswerten einen möglichst steilen Anstieg, so daß sich für niedrige Konzentrationen ein möglichst hohes Signal-Rauschverhältnis ergibt. Da der maximal als Meßsignal mögliche Strom begrenzt ist, bewirken Sättigungseffekte bei höheren Konzentrationen einen konstanten oder nahezu konstanten Maximalstrom Iₗᵢₘ. Im Gegensatz dazu hat die Kennlinie 20 der ersten Meßelektrode einen möglichst linearen Verlauf, so daß sich auch hohe Analytkonzentrationen ohne den Einfluß von Sättigungseffekten zuverlässig bestimmen lassen. Während bei der ersten Kennlinie 20 eine Auswertung von kleinen Meßströmen I, die mit niedrigen Konzentrationen korrespondieren, durch ein ungünstiges Signal-Rauschverhältnis erschwert ist, lassen sich mit der zweiten Kennlinie 40 Meßströme I, die einen Schwellenwert Iₗᵢₘ übersteigen, wegen Sättigungseffekten nicht sinnvoll auswerten.

In einem Arbeitsschritt 50 wird deshalb geprüft, ob der Meßstrom I der zweiten Meßelektrode kleiner als ein vorgegebener Schwellenwert Iₗᵢₘ ist. Falls dies der Fall ist, ist ein durch Auswertung des Meßsignals der zweiten Meßelektrode gewonnener Konzentrationswert C_{H} zuverlässiger als der anhand der ersten Meßelektrode bestimmte Konzentrationswert C_{F}, so daß der Konzentrationswert C_{H} als Ergebnis ausgegeben wird. Falls jedoch der Meßstrom der zweiten Meßelektrode größer oder gleich dem Schwellenwert Iₗᵢₘ ist, ist der anhand der ersten Meßelektrode bestimmte Konzentrationswert C_{F} zuverlässiger als der anhand der zweiten Meßelektrode bestimmte Konzentrationswert C_{H}, so daß der Wert C_{F} als Konzentrationswert ausgegeben wird.

Prinzipiell kann auf den Arbeitsschritt 50 verzichtet werden, wenn der in dem Arbeitsschritt 30 geprüfte Schwellenwert, der bei dem dargestellten Beispiel 80 mg/dl beträgt, geeignet gewählt wird. Nach der Implantation eines Elektrodensystems kann es jedoch zu Änderungen der Meßsensitivität der einzelnen Meßelektroden kommen, so daß der Sättigungsbereich der zweiten Meßelektrode schon bei kleineren Konzentrationen beginnt. In dem Arbeitsschritt 50 kann dies erkannt werden und gegebenenfalls der Konzentrationsbereich, in dem Analytkonzentrationen mit der ersten Meßelektrode bestimmt werden, etwas zu tieferen Analytkonzentrationen hin ausgedehnt werden. Möglich ist es auch, den Konzentrationsbereich für den die erste Meßelektrode optimiert ist, und den Konzentrationsbereich für den die zweite Meßelektrode optimiert ist, überlappend zu wählen und in einem Überlappungsbereich die Analytkonzentration mittels einer statistischen Auswertung aus einem Auswerteergebnis der ersten Meßelektrode und einem Auswertungsergebnis der zweiten Meßelektrode zu ermitteln. Beispielsweise kann der Überlappungsbereich von 70 mg/dl bis 100 mg/dl gewählt werden. Die statistischen Gewichte, mit denen die Ergebnisse der ersten und der zweiten Meßelektrode in dem Überlappungsbereich gewichtet werden, können unter Berücksichtigung des Signal-Rauschverhältnisses des jeweiligen Meßsignals gewählt werden.

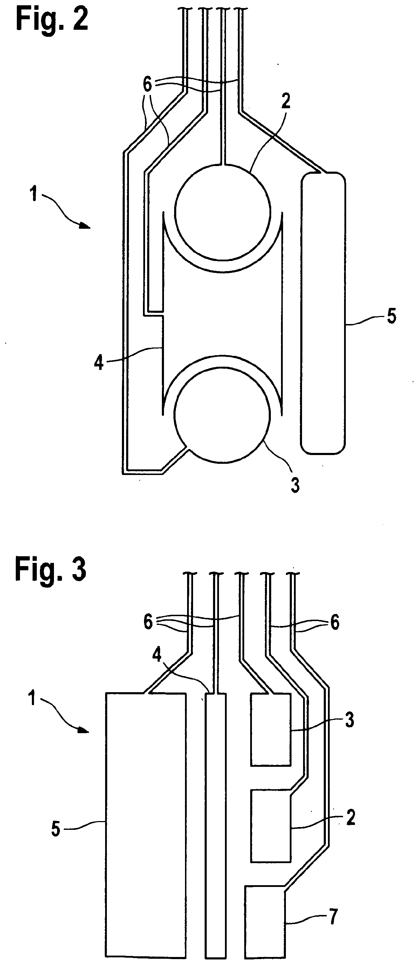

In Figur 2 ist ein Ausführungsbeispiel eines Elektrodensystems 1 dargestellt, mit dem sich das beschriebene Verfahren durchführen läßt. Das Elektrodensystem 1 umfaßt eine erste Meßelektrode 2 und eine zweite Meßelektrode 3, die jeweils unterschiedliche Meßsensitivitäten haben. Den beiden Meßelektroden 2, 3 ist eine gemeinsame Gegenelektrode 4 zugeordnet, die im Betrieb auf Massepotential gelegt wird, so daß zwischen der erste Meßelektrode 2 und der Gegenelektrode 4 ein erster Meßstrom I1 und zwischen der zweiten Meßelektrode 3 und der Gegenelektrode 4 ein zweiter Meßstrom I2 fließt. Das Elektrodensystem 1 umfaßt ferner eine Referenzelektrode 5, die ein Bezugspotential für die Meßelektroden 2, 3 liefert. Das Bezugspotential wird bevorzugt durch die Redoxreaktion Silber/Silberchlorid definiert, wobei selbstverständlich auch andere Redoxreaktionen für die Referenzelektrode genutzt werden können.

Prinzipiell kann auf einem separate Referenzelektrode 5 verzichtet werden und die Gegenelektrode 4 zugleich als Referenzelektrode genutzt werden. Da durch die Gegenelektrode 4 jedoch die Meßströme I1 und I2 fließen, würde dann die das Referenzpotential definierende Redoxreaktion (beispielsweise Silber/Silberchlorid) mit der Zeit zum Erliegen kommen, so daß die Lebensdauer des Elektrodensystems begrenzt wäre. Mit einer separaten Referenzelektrode 5 kann das Referenzpotential stromlos zur Verfügung gestellt werden, so daß sich in dieser Hinsicht keine Beschränkung für die Lebensdauer des Elektrodensystems ergibt.

In Figur 3 ist ein weiteres Ausführungsbeispiel eines implantierbaren Elektrodensystems 1 dargestellt, das sich von dem in Figur 2 dargestellten Ausführungsbeispiel abgesehen von Form und Anordnung der einzelnen Elektroden dadurch unterscheidet, daß insgesamt drei Meßelektroden 2, 3, 7 vorhanden sind. Ähnlich wie bei dem in Figur 2 dargestellten Elektrodensystem 1 dient die zweite Meßelektrode 3 dazu, Glukosekonzentrationen unterhalb von 80 mg/dl zu messen. Während bei dem in Figur 2 dargestellten Ausführungsbeispiel die erste Meßelektrode 2 für den gesamten Konzentrationsbereich oberhalb von 80 mg/dl verwendet wird, ist bei dem in Figur 3 dargestellten Elektrodensystem 1 eine dritte Meßelektrode 7 für Konzentrationen von mehr als 300 mg/dl vorhanden.

Insbesondere bei Verwendung von mehr als zwei Meßelektroden ist es günstig, die Meßsensitivitäten der einzelnen Meßelektroden so zu wählen, daß sich überlappende Meßbereiche ergeben. Überlappt beispielsweise der erste Konzentrationsbereich, für den die Meßsensitivität der ersten Meßelektrode 2 optimiert ist, mit dem zweiten Konzentrationsbereich, für den die zweite Meßelektrode 3 optimiert ist, sind in dem Überlappungsbereich Plausibilitätsprüfungen möglich. Ferner können Analytkonzentrationen in dem oder den Überlappungsbereichen mittels einer statistischen Auswertung aus Auswertungsergebnissen von verschiedenen Meßelektroden ermittelt werden.

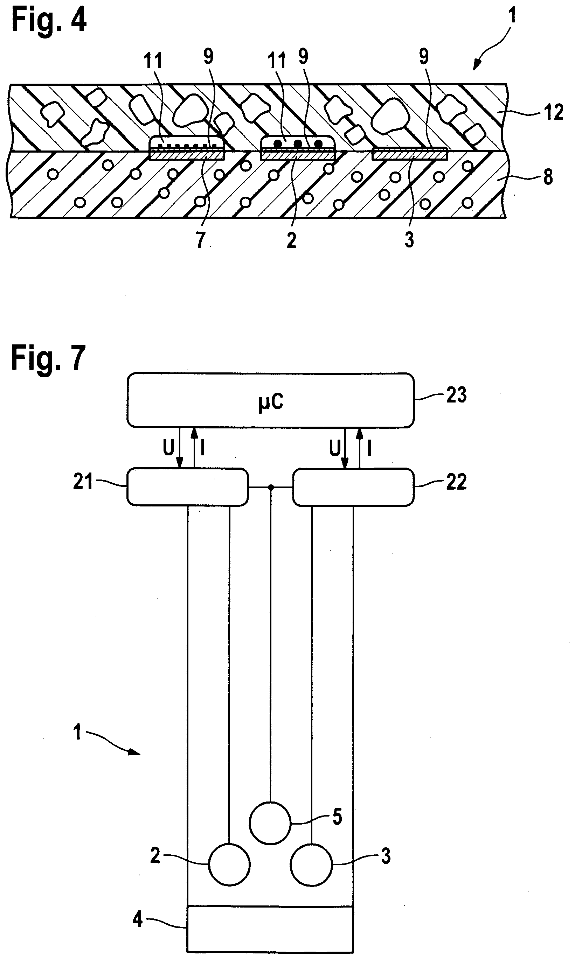

In Figur 4 ist das in Figur 3 dargestellte Elektrodensystem in einem Querschnitt dargestellt, wobei die Schnittlinie durch die Meßelektroden 2, 3 und 7 verläuft. Die Elektroden 2, 3, 4, 5 und 7 sind auf einem gemeinsamen Träger 8, der bevorzugt aus Kunststoff, beispielsweise aus Polyimid besteht, angeordnet und durch Leiterbahnen 6 kontaktiert, über die das Elektrodensystem 1 an eine Auswerteeinheit (nicht dargestellt) angeschlossen werden kann. Im Betrieb stellt die Auswerteeinheit das Potential der Meßelektroden 2, 3, 7 auf einen vorgegebenen Wert von beispielsweise 350 mV gegenüber der Referenzelektrode 5 ein. Obwohl die Meßelektroden, beispielsweise durch Anlagerungen von Proteinen nach Implantation gegenüber dem Massepotential der Gegenelektrode 4 driften, können auf diese Art und Weise definierte Verhältnisse für eine präzise Auswertung der Meßströme geschaffen werden, da davon ausgegangen werden kann, daß die Meßelektroden 2, 3, 7 in gleicher Weise wie die Referenzelektrode 5 Drifteffekten unterliegen.

Die Meßelektroden 2, 3, 7 haben jeweils eine Fläche von 0,1 mm² bis 0,5 mm² und bestehen ebenso wie die Leiterbahnen 6 aus Gold. Die Meßelektroden 2, 3, 7 sind jeweils mit einer Enzymschicht 9 belegt, die ein Enzym enthält, das durch katalytische Umwandlung des Analyten Ladungsträger erzeugt, die für das Meßsignal erfaßt werden. Die Ladungsträger können entweder direkt oder durch Umsetzung eines Zwischenprodukts, das von dem Enzym zunächst gebildet wird, erzeugt werden. Ein Beispiel für ein derartiges Zwischenprodukt ist Wasserstoffperoxid. Bei dem dargestellten Ausführungsbeispiel sind die Enzymschichten 9 als Karbonschichten ausgebildet, die das Enzym Glukoseoxidase immobilisieren. Die Enzymschichten haben eine Dicke von 3 um bis 10 µm, bevorzugt 5 µm.

Die unterschiedlichen Meßsensitivitäten der Meßelektroden 2, 3, 7 werden erfindungsgemäß dadurch erzeugt, daß mindestens eine der Meßelektroden 2, 3, 7 eine Deckschicht 11 aufweist, die dem Analyten einen Diffusionswiderstand entgegensetzt, wobei durch Anpassung des Diffusionswiderstandes ein Unterschied zwischen den Meßsensitivitäten der Meßelektroden 2, 3 und 7 realisiert ist. Da der Diffusionswiderstand einer Deckschicht 11 um so größer ist, je dicker die betreffende Deckschicht 11 ist, lassen sich unterschiedliche Diffusionswiderstände am einfachsten durch unterschiedlich dicke Deckschichten 11 realisieren. Beispielsweise kann hierfür auf der ersten Meßelektrode 2 eine 30 µm dicke Deckmembran aus einem schwachquellenden Polymer, beispielsweise Polyurethan und auf der zweiten Meßelektrode 3 eine nur 10 µm starke Deckmembran aus dem selben Material aufgebracht werden. Bedingt durch unterschiedliche Diffusionswiderstände der Deckschichten der Meßelektroden 2, 3 erreichen pro Zeiteinheit unterschiedlich viele Glukosemoleküle, die Enzymschichten der Meßelektroden 2, 3. Von dem in der Enzymschicht gespeichertem Enzym Glukoseoxidase werden Glukosemoleküle abgebaut, so daß Ladungsträger frei werden, die an den Meßelektroden 2, 3 erfaßt werden und auf diese Weise die Meßströme I1 und I2 bilden. Unterschiedliche Diffusionswiderstände können im einfachsten Fall durch eine unterschiedliche Dicke der Deckschichten der Meßelektroden 2, 3 realisiert werden.

Eine weitere Möglichkeit besteht darin, die Mikrostruktur der Deckschichten, beispielsweise deren Porosität, unterschiedlich zu gestalten oder die Deckschichten aus unterschiedlichen Materialien herzustellen. Beispielsweise kann für die Deckschicht 11 der Meßelektrode 7 für hohe Konzentrationen Silikon verwendet werden, das für Glukosemoleküle relativ undurchlässig ist, für die Deckschicht 11 der Meßelektrode 3 für mittlere Konzentrationen Polyurethan verwendet werden, das für Glukosemoleküle relativ durchlässig ist und für die Meßelektrode 2 auf eine Deckschicht verzichtet werden.

Für die Deckschicht 11 sind schwachquellende Polymere, wie beispielsweise Polyurethan besonders günstig. Die Dicke der Deckmembran beträgt bevorzugt weniger als 50 µm, besonders bevorzugt 10 µm bis 30 µm.

Bedingt durch die unterschiedlichen Diffusionswiderstände der Deckschichten 11 der Meßelektroden 2, 3 erreichen pro Zeiteinheit unterschiedlich viele Glukosemoleküle, die Enzymschichten der Meßelektroden 2, 3. Von dem in der Enzymschicht gespeichertem Enzym Glukoseoxidase werden die Glukosemoleküle abgebaut, so daß Ladungsträger frei werden, die an den Meßelektroden 2, 3 erfaßt werden und auf diese Weise unterschiedliche Meßströme bilden.

Eine weitere Möglichkeit unterschiedliche Meßsensitivitäten der ersten und der zweiten Meßelektrode 2, 3 zu realisieren, besteht darin, unterschiedliche Enzymschichten 9 einzusetzen. Beispielsweise kann zur Erhöhung der Meßsensitivität der ersten Meßelektrode 2 die Enzymmenge in der Enzymschicht 9 doppelt so groß gewählt werden, wie für die Enzymschicht 9 der zweiten Meßelektrode 3.

Die Meßelektroden 2, 3, 7 sind ferner von einer Dialysemembran 12 bedeckt, die sich bevorzugt über die gesamte Oberfläche des Elektrodensystems 1 erstreckt. Unter einer Dialysemembran 12 wird in diesem Zusammenhang eine Membran verstanden, die für Moleküle oberhalb einer Maximalgröße undurchlässig ist. Die Dialysemembran 12 wird in einem separaten Herstellungsprozeß vorgefertigt und als vollständige, bestehende Struktur bei der Fertigung des Elektrodensystems 1 aufgebracht. Diese Maximalgröße der Dialysemembran wird für das dargestellte Elektrodensystem 1 so gewählt, daß Analytmoleküle durch die Dialysemembran 12 hindurch treten können, größere Moleküle jedoch abgehalten werden. Beim dem dargestellten Ausführungsbeispiel ist die Dialysemembran 12 als eine poröse Schicht aus einem geeignetem Kunststoff, insbesondere Polyarylethersulfon, ausgebildet. Durch eine Dialysemembran 12 läßt sich die effektive Oberfläche der Meßelektroden 2, 3, 7 vergrößern und folglich das Signal-Rauschverhältnis verbessern. Prinzipiell kann auf eine Dialysemembran jedoch verzichtet werden, sofern alle Meßelektroden mit einer Deckschicht 11 versehen sind.

Wichtig für ein zuverlässiges Funktionieren des beschriebenen Elektrodensystems 1 ist, daß sämtliche Meßelektroden 2, 3, 7 der selben Analytkonzentration ausgesetzt sind. Bevorzugt sind die einzelnen Meßelektroden 2, 3, 7 deshalb weniger als 1,5 mm, bevorzugt weniger als 1 mm, besonders bevorzugt weniger als 700 µm voneinander entfernt. Werden mehrere Meßelektroden verwendet, ist deshalb darauf zu achten, daß auch zwischen den am weitesten voneinander entfernten Meßelektroden keine zu großen Abstände auftreten. Im Bereich des Elektrodensystems 1 lassen sich homogene Analytkonzentrationen, beispielsweise durch eine ausreichend dicke Dialysemembran bewirken. Bevorzugt hat die Dialysemembran deshalb eine Dicke von 50 µm bis 500 µm, besonders bevorzugt 100 µm bis 300 µm.

Eine weitere Möglichkeit, die Analytkonzentrationen im Bereich des Elektrodensystems 1 zu homogenisieren besteht darin, die Dialysemembran 12 nicht unmittelbar auf den Meßelektroden 2, 3, 7 anzuordnen, sondern die Meßelektroden 2, 3, 7 in einer kleinen Kammer anzuordnen, die von der Dialysemembran verschlossen ist. Eine derartige Kammer läßt sich beispielsweise dadurch verwirklichen, daß die Meßelektroden 2, 3, 7 in einer geeigneten Vertiefung des Trägers 8 angeordnet werden und die Vertiefung von der Dialysemembran 12 bedeckt wird. Die Höhe einer solchen Kammer, d. h. der Abstand von der Oberfläche der Meßelektroden 2, 3, 7 (bzw. deren Deckschicht 11) bis zu der Unterseite der Dialysemembran 12 beträgt bevorzugt weniger als 400 µm, besonders bevorzugt weniger als 300 µm.

Eine derartige Kammer hat ebenso wie eine dicke Dialysemembran den Vorteil als Reservoir für den Analyten zu dienen, so daß eine vorübergehende lokale Blockade der Dialysemembran 12 ausgeglichen werden kann.

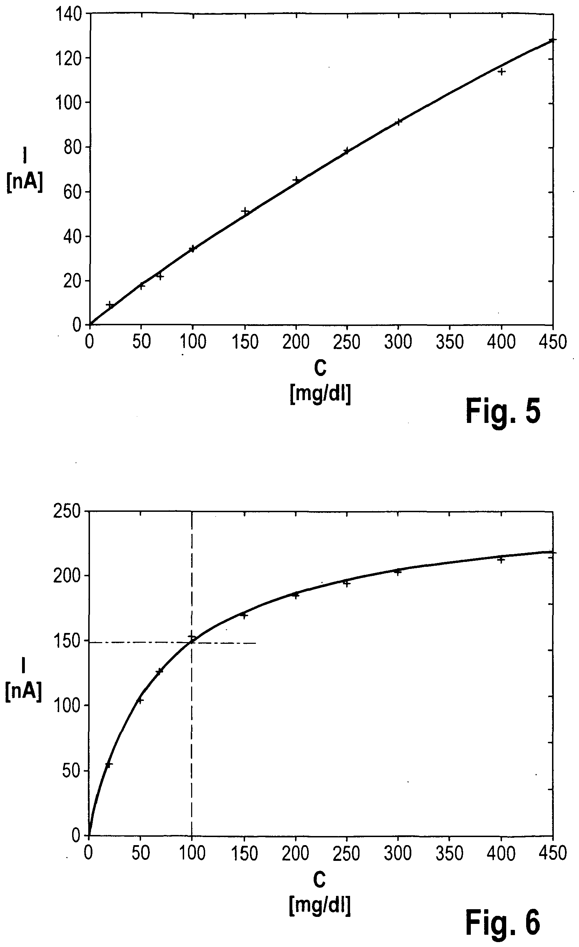

In Figur 5 ist die im Zusammenhang von Figur 1 bereits erläutere Kennlinie 20 im Detail dargestellt, welche die funktionale Abhängigkeit des das Meßsignal der ersten Meßelektrode 2 darstellenden Stroms I von der Analytkonzentration C darstellt. In der Praxis wird die Meßgenauigkeit bei der Bestimmung hoher Analytkonzentrationen durch Sättigungseffekte beschränkt. Um die Meßsensitivität der ersten Meßelektrode für hohe Analytkonzentrationen zu optimieren, ist es deshalb günstig, über den gesamten physiologisch relevanten Konzentrationsbereich einen möglichst linearen Verlauf der Kennlinie anzustreben.

Im menschlichen Körper kommen Glukosekonzentrationen von mehr als 450 mg/dl praktisch nicht vor, so daß der Verlauf der in Figur 5 dargestellten Kennlinie oberhalb von 450 mg/dl ohne Belang ist. Ist der Zusammenhang zwischen Strom I und Konzentration C linear, so läßt sich die Sensitivität der Meßelektrode durch einen einzigen Sensitivitätsparameter charakterisieren, der die Ableitung der Kennlinie nach der Konzentration, d. h. die Steigung ist. In der Praxis läßt sich ein perfekt linearer Zusammenhang meist nicht realisieren, so daß zusätzliche Sensitivitätsparameter benötigt werden, um den Verlauf der Kennlinie zu beschreiben.

Damit der Einfluß von Sättigungseffekten möglichst vernachlässigbar ist, sollte die Sensitivität der ersten Meßelektrode 2 bei 450 mg/dl mindestens 80 % der Sensitivität bei 100 mg/dl betragen, wie dies bei in Figur 5 dargestellten Kennlinie der Fall ist. Des weiteren sollte die Sensitivität bei 100 mg/dl mindestens 0,1 nA/mg/dl betragen, so daß Konzentrationen oberhalb von 100 mg/dl mit einem ausreichend hohen Signal-Rauschverhältnis erfaßt werden können. Für Konzentrationen unterhalb von etwa 100 mg/dl führt die in Figur 5 dargestellte Kennlinie zu einem zunehmend ungünstigen Signal-Rauschverhältnis, so daß die Meßgenauigkeit für niedrige Konzentrationen unzureichend ist.

Zur Messung von Analytkonzentrationen unterhalb von 80 mg/dl wird deshalb eine zweite Meßelektrode verwendet, die im diesem niedrigen Konzentrationsbereich eine wesentlich höhere Sensitivität hat. Ein Beispiel der Kennlinie einer geeigneten Meßelektrode ist in Figur 6 dargestellt. Eine möglichst hohe Meßsensitivität bei niedrigen Konzentrationen bedeutet eine möglichst steile Kennlinie in dem entsprechenden Konzentrationsbereich. Da die mit implantierten Elektroden erzielbaren Meßströme begrenzt sind, ist eine hohe Meßsensitivität bei niedrigen Konzentrationen mit einer Sättigung bei höheren Konzentrationen verbunden. Dies zeigt sich in Figur 6 an einem deutlichen Abflachen der Kennlinie bei Konzentrationen von mehr als 200 mg/dl. Für hohe Konzentrationen ergibt sich deshalb ein sehr ungünstiges Signalrauschverhältnis, dem jedoch ein sehr günstiges Signalrauschverhältnis bei tiefen Konzentrationen gegenübersteht.

Die Meßsensitivitäten der ersten Meßelektrode und der zweiten Meßelektrode unterscheiden sich bei einer Referenzkonzentration, beispielsweise einer Glucosekonzentration von 100 mg/dl, mindestens um einen Faktor 2, vorzugsweise mindestens um einen Faktor 3. Als Referenzkonzentration wird bevorzugt eine Konzentration aus dem ersten oder dem zweiten Konzentrationsbereich gewählt, beispielsweise das arithmetische Mittel aus der Obergrenze und der Untergrenze eines der Konzentrationsbereiche. Unter der Meßsensitivität ist dabei die Ableitung der Intensität des Meßsignals nach der Konzentration, d. h. die Steigung der Kennlinie bei der betreffenden Konzentration, zu verstehen.

Damit kritische Glukosekonzentrationen von 50 mg/dl mit der zweiten Meßelektrode zuverlässig erkannt werden können, sollte die Sensitivität der zweiten Meßelektrode zwischen 10 mg/dl und 100 mg/dl mindestens 1 nA/mg/dl betragen. Auf diese Weise ergeben sich bei einer kritischen Glukosekonzentration von 50 mg/dl Ströme von mindestens 50 nA, so daß ein Signal-Rauschverhältnis von 5 oder besser erreicht werden kann.

In Figur 7 ist eine schematische Darstellung einer erfindungsgemäßen Vorrichtung zum Messen einer Analytkonzentration mit einem erfindungsgemäßen Elektrodensystem 1 dargestellt. Dabei ist das im vorhergehenden beschriebene Elektrodensystem 1 an eine Auswerteeinheit 23 in Form eines Mikroprozessors angeschlossen, die einen Speicher umfaßt, in dem mindestens ein die Meßsensitivität der ersten Meßelektrode 2 charakterisierender erster Sensitivitätsparameter und mindestens ein die Meßsensitivität der zweiten Meßelektrode 3 charakterisierender zweiter Sensitivitätsparameter gespeichert sind. Die Auswerteeinheit 23 stellt durch Auswertung mindestens eines Meßsignal einer der Meßelektroden 2, 3 fest, in welchem Konzentrationsbereich die Analytkonzentration in der Umgebung des Elektrodensystems liegt. Liegt die Analytkonzentration in dem Konzentrationsbereich, für den die Meßsensitivität der ersten Meßelektrode 2 optimiert ist, wird die Analytkonzentration durch Auswertung des Meßsignals der ersten Meßelektrode 2 ermittelt. Liegt die Analytkonzentration in dem Konzentrationsbereich, für den die Meßsensitivität der zweiten Meßelektrode 3 optimiert ist, wird die Analytkonzentration durch Auswertung des Meßsignals der zweiten Meßelektrode bestimmt.

Falls die Konzentrationsbereiche der einzelnen Meßelektroden 2, 3 überlappen, können Analytkonzentrationen in einem Überlappungsbereich auch durch Auswertung der Meßsignale von zwei Meßelektroden 2, 3 ermittelt werden.

Die erste Meßelektrode 2 ist an einen ersten Potentiostaten 21 und die zweite Meßelektrode 3 an einen zweiten Potentiostaten 22 angeschlossen. Die Potentiostaten 21, 22 werden jeweils von dem die Steuer- und Auswerteeinheit 23 bildenden Mikroprozessor gesteuert. Ein Potentiostat ist ein elektronischer Regelkreis, mit dem das an der betreffenden Meßelektrode 2, 3 anliegende Potential bezüglich einer Referenzelektrode 5 auf einen gewünschten Wert geregelt wird. Zwischen den Meßelektroden 2, 3, an denen die enzymatische Umsetzung des Analyten einschließlich Ladungsträgererzeugung bei entsprechender Potentialeinregelung erfolgt, und der Gegenelektrode 4 fließt der zu messende Strom. Der Referenzpunkt wird durch die Referenzelektrode 5 dargestellt, deren Potential in der elektrochemischen Spannungsreihe definiert ist. Durch die Referenzelektrode 5 selbst fließt bevorzugt kein Strom.

Bei einem implantierten Elektrodensystem ist der maximale Meßstrom sehr begrenzt, da die für den Meßstrom erforderlichen Ladungsträger durch enzymatischen Abbau des Analyten erzeugt werden. Durch den Einfluß des Meßstroms und des verwendeten Enzyms droht deshalb die Analytkonzentration in der für die Messung relevanten Umgebung des Elektrodensystems 1 zu verarmen, insbesondere wenn das die Meßelektroden 2, 3 umgebende Körpergewebe nur relativ schwach von Körperflüssigkeit durchströmt wird, oder der Transport von Analytmolekülen in die Umgebung der Meßelektroden 2, 3 aus anderen Gründen, beispielsweise durch eine Blockade, gehemmt ist. Die Analytmenge, die von einer Meßelektrode im kontinuierlichen Meßbetrieb pro Minute typischerweise verbraucht wird, liegt im Pikomol-Bereich, ist also relativ klein. Dennoch kann durch die Verarmung der Analytkonzentration im Bereich der Meßelektroden 2, 3 eine Verfälschung der Messung auftreten. Im ungünstigsten Fall kann dieser Effekt zu einem physiologisch plausiblen Signalabfall führen, also eine Abnahme der Analytkonzentration im gesamten Körper des Patienten vortäuschen.

Mit der in Figur 7 dargestellten Vorrichtung läßt sich prüfen, ob ein mit dem Elektrodensystem 1 über einen bestimmten Zeitraum beobachteter Abfall der Glukosekonzentration eine natürliche Ursache hat und folglich im gesamten Körper des Patienten auftritt oder nur lokal in der Umgebung des Elektrodensystems auftritt. Ein nicht physiologischer Beitrag zu einem Signalabfall, der durch eine lokale Verarmung der Glukosekonzentration in der Umgebung der Meßelektroden 2, 3 verursacht wird, wird dadurch erkannt, daß an die Meßelektroden 2, 3 alternierend eine Meßspannung angelegt wird. Werden die Meßelektroden 2, 3 innerhalb eines zur Erkennung der Verarmung relevanten Zeitraums von mehreren Minuten getrennt an- und abgeschaltet, kann für jede Meßelektrode der Signalverlauf untersucht werden. Wird dabei für die Meßelektrode mit der höheren Meßsensitivität ein schnellerer Signalabfall festgestellt als für die Meßelektrode mit der niedrigeren Meßsensitivität, deutet dies auf eine Verarmung hin.

Durch einen einfachen Algorithmus, beispielsweise eine lineare Ausgleichsrechnung oder eine nichtlineare Kurvenauswertung, können für die Meßströme der Meßelektroden 2, 3 jeweils charakteristische Abfallzeiten bestimmt werden, wobei ein sich einstellender Gleichgewichtszustand numerisch berücksichtigt werden kann. Werden dabei von der Auswerteeinheit 23 innerhalb eines für die Verarmung relevanten Zeitraums für die Meßelektroden 2, 3 unterschiedliche charakteristische Abfallzeiten festgestellt, kann durch ein Alarmsignal auf die Unzuverlässigkeit der errechneten Glukosekonzentration hingewiesen werden.

Sofern die festgestellte Verarmung einen kritischen Schwellenwert nicht übersteigt, besteht die Möglichkeit, die durch den Meßstrom bewirkte Abnahme der Analytkonzentration numerisch bei der Auswertung der Meßströme zur Bestimmung der realen Analytkonzentration in dem Körper des Patienten zu kompensieren. Beispielsweise kann die Verbrauchskinetik der Ladungsträger in bezug auf den Analyten durch ein einfaches Modell erfaßt werden. Die von der Auswerteeinheit 23 auf der Grundlage der aktuellen Messungen ermittelten Abfallzeiten können mit theoretischen Werten des Modells verglichen werden und die Grundlage für eine numerische Kompensation der aus den Meßwerten ermittelten lokalen Analytkonzentration liefern.

Bei dem dargestellten Ausführungsbeispiel führt die Auswerteeinheit 23 also bei der Auswertung der Meßsignale ein Aufbereitungsverfahren durch. Bei diesem Verfahren werden Korrekturdaten, die eine durch einen Meßstrom bewirkte lokale Abnahme der Analytkonzentration betreffen, berücksichtigt, wobei zu der Gewinnung der Korrekturdaten die an den Meßelektroden 2, 3 anliegenden Meßspannungen separat an- und abgeschaltet werden und der zeitliche Verlauf der Meßströme ausgewertet wird, insbesondere indem charakteristische Abfallzeiten der Meßströme bestimmt werden. Im einfachsten Fall kann es sich bei diesen Korrekturdaten um die charakteristischen Abfallzeiten selbst handeln.

Bei dem in Figur 7 dargestellten Ausführungsbeispiel bildet der Mikroprozessor 23 sowohl die Auswerteeinheit zum Auswerten der Meßsignale als auch die Steuereinheit, mit der die jeweils an den Meßelektroden 2,3 anliegenden Meßspannungen separat an- und abschaltbar sind. Die Auswerteeinheit 23 ist derart ausgebildet, daß eine nach dem Einschalten der Meßspannung für mindestens eine der Meßelektroden 2, 3 durch einen Meßstrom bewirkte lokale Abnahme der Analytkonzentration erkannt und bei der Auswertung zur Bestimmung der Analytkonzentration in dem menschlichen oder tierischen Körper berücksichtigt wird. Diese Berücksichtigung kann in der Weise erfolgen, daß durch ein Signal angezeigt wird, daß eine Bestimmung der Analytkonzentration derzeit nicht möglich ist. Eine weitere Möglichkeit besteht darin, die lokale Abnahme der Analytkonzentration quantitativ zu erfassen und numerisch bei der Berechnung der Analytkonzentration in dem Körper des Patienten zu kompensieren.

Die Auswerte- und Steuereinheit 23 kann beispielsweise auch zur Steuerung einer künstlichen Bauchspeicheldrüse dienen oder an eine Anzeigeeinrichtung angeschlossen sein, mit der ermittelte Analytkonzentrationen angezeigt oder/und ein Patient vor besonders hohen oder tiefen Analytkonzentrationen, die Gegenmaßnahmen erfordern, durch ein geeignetes Signal, beispielsweise ein akustisches Signal, gewarnt wird. Besonders günstig ist es in diesem Zusammenhang, wenn die Datenübertragung zu der Anzeigeeinrichtung drahtlos, beispielsweise durch eine Infrarotschnittstelle oder mittels Ultraschall, erfolgt.

## Patentansprüche

1. Implantierbares Elektrodensystem für eine Vorrichtung zum Messen einer Analytkonzentration in einem menschlichen oder tierischen Körper, umfassend
eine erste und eine zweite Meßelektrode (2, 3), um jeweils Meßsignale zu ermitteln, die eine Information über die zu messende Analytkonzentration enthalten, wobei
die Meßelektroden (2, 3, 7) jeweils eine Enzymschicht (9) mit einem Enzym aufweisen, das durch katalytische Umwandlung des Analyten Ladungsträger erzeugt, die für das Meßsignal erfaßt werden,
die erste Meßelektrode (2) eine erste Meßsensitivität hat, die an einen ersten Konzentrationsbereich des Analyten angepaßt ist,
die zweite Meßelektrode (3) eine zweite Meßsensitivität hat, die von der ersten Meßsensitivität verschieden ist und an einen zweiten Konzentrationsbereich des Analyten angepaßt ist, **dadurch gekennzeichnet, daß** die Meßelektroden (2, 3, 7) eine gemeinsame Gegenelektrode (4) haben, die Teil des Elektrodensystems ist, und
daß mindestens eine der Meßelektroden (2, 3, 7) eine Deckschicht (11) aufweist, die dem Analyten einen Diffusionswiderstand entgegensetzt, wobei durch Anpassung des Diffusionswiderstands ein Unterschied zwischen den Meßsensitivitäten der ersten Meßelektrode (2) und der zweiten Meßelektrode (3) realisiert ist.

2. Elektrodensystem nach Anspruch 1, **dadurch gekennzeichnet, daß** sich die Meßsensitivitäten der Meßelektroden (2, 3) bei einer Referenzkonzentration mindestens um einen Faktor 2, bevorzugt mindestens um einen Faktor 3 unterscheiden.

3. Elektrodensystem nach einem der vorstehenden Ansprüche, **dadurch gekennzeichnet, daß** die unterschiedlichen Meßsensitivitäten der ersten und der zweiten Meßelektrode (2, 3) durch unterschiedliche Enzymschichten (9) mit unterschiedlichen Enzymen und/oder unterschiedlichen Enzymmengen realisiert sind.

4. Elektrodensystem nach einem der vorstehenden Ansprüche, **dadurch gekennzeichnet, daß** es mindestens eine weitere Meßelektrode (7) umfaßt, deren Meßsensitivität an einen weiteren Konzentrationsbereich des Analyten angepaßt ist.

5. Vorrichtung zum Messen einer Analytkonzentration in einem menschlichen oder tierischen Körper, umfassend:
ein implantierbares Elektrodensystem (1) nach einem der vorstehenden Ansprüche und
eine an das Elektrodensystem (1) angeschlossene Auswerteeinheit zum Auswerten von Meßsignalen der ersten und der zweiten Meßelektrode (2, 3),
**gekennzeichnet durch**,
einen Speicher, in dem mindestens ein die Meßsensitivität der ersten Meßelektrode (2) charakterisierender erster Sensitivitätsparameter und mindestens ein die Meßsensitivität der zweiten Meßelektrode (3) charakterisierende zweiter Sensitivitätsparameter gespeichert sind,
wobei die Auswerteeinheit (23) derart ausgebildet ist, daß **durch** Auswertung mindestens eines Meßsignals einer der Meßelektroden (2, 3) feststellbar ist, in welchem Konzentrationsbereich die Analytkonzentration liegt, und
falls die Analytkonzentration in dem ersten Konzentrationsbereich liegt, das Meßsignal der ersten Meßelektrode (2) mittels des ersten Sensitivitätsparameter zum Bestimmen der Analytkonzentration ausgewertet wird, und
falls die Analytkonzentration in dem zweiten Kon zentrationsbereich liegt, das Meßsignal der zweiten Meßelektrode (3) mittels des zweiten Sensitivitätsparameter zum Bestimmen der Analytkonzentration ausgewertet wird.

6. Vorrichtung nach Anspruch 5, **dadurch gekennzeichnet, daß** der erste Konzentrationsbereich, für den die erste Meßelektrode (2) optimiert ist, und der zweite Konzentrationsbereich, für den die zweite Meßelektrode (3) optimiert ist, in einem Überlappungsbereich überlappen, und
die Auswerteeinheit (23) so ausgebildet ist, daß sowohl das Meßsignal der ersten Meßelektrode (2) als auch das Meßsignal der zweiten Meßelektrode (3) jeweils ausgewertet werden, um festzustellen in welchem oder welchen Konzentrationsbereichen die Analytkonzentration liegt, und in dem Überlappungsbereich liegende Analytkonzentrationen mittels einer statistischen Auswertung aus einem Auswertungsergebnis der ersten Meßelektrode (2) und einem Auswertungsergebnis der zweiten Meßelektrode (3) ermittelt werden.

7. Vorrichtung nach Anspruch 5 oder 6, **dadurch gekennzeichnet, daß** die erste Meßelektrode (2) an einen ersten Potentiostaten (21) und die zweite Meßelektrode (3) an einen zweiten Potentiostaten (22) angeschlossen ist.

8. Vorrichtung nach einem der Ansprüche 5 bis 7, **dadurch gekennzeichnet, daß** die jeweils an den Meßelektroden (2, 3) anliegenden Meßspannungen mittels einer Steuereinheit (23) separat an- und abschaltbar sind, wobei die Auswerteeinheit (23) derart ausgebildet ist, daß eine nach dem Einschalten der Meßspannung für mindestens eine der Meßelektroden (2, 3) durch einen Meßstrom bewirkte lokale Abnahme der Analytkonzentration erkannt und bei der Auswertung zur Bestimmung der Analytkonzentration in dem menschlichen oder tierischen Körper berücksichtigt, vorzugsweise numerisch kompensiert, wird.

## Claims

1. Implantable electrode system for a device for measuring an analyte concentration in a human or animal body, comprising
a first and a second measuring electrode (2, 3) for determining measuring signals which each contain information concerning the analyte concentration to be measured, wherein
the measuring electrodes (2, 3, 7) each comprise an enzyme layer with an enzyme that creates charge carriers by catalytic conversion of the analyte,
the first measuring electrode (2) has a first measuring sensitivity that is adapted to a first concentration range of the analyte, and
the second measuring electrode (3) has a second measuring sensitivity that differs from the first measuring sensitivity and is adapted to a second concentration range of the analyte,
**characterized in**
**that** the measuring electrodes (2, 3, 7) have a common counter-electrode (4) that is part of the electrode system, and
**that** at least one of the measuring electrodes (2, 3, 7) comprises a covering layer (11) that produces a diffusion resistance for the analyte, whereby a difference between the measuring sensitivities of the first measuring electrode (2) and the second measuring electrode (3) is implemented by an adaption of the diffusion resistance.

2. Electrode system according to claim 1, **characterized in that** the measuring sensitivities of the measuring electrodes (2, 3) differ at a reference concentration by at least a factor of 2, preferably by at least a factor of 3.

3. Electrode system according to any one of the preceding claims, **characterized in that** the different measuring sensitivities of the first and the second measuring electrode (2, 3) are implemented by different enzyme layers (9) with different enzymes and/or different quantities of enzyme.

4. Electrode system according to any one of the preceding claims, **characterized in that** it comprises at least one further measuring electrode (7) whose measuring sensitivity is adapted to a further concentration range of the analyte.

5. Device for measuring an analyte concentration in a human or animal body, comprising:
an implantable electrode system (1) according to any one of the preceding claims,
an analytical unit connected to the electrode system (1) for analyzing measuring signals of the first and the second measuring electrode (2, 3),
**characterized by**
a memory, in which at least one first sensitivity parameter characterizing the measuring sensitivity of the first measuring electrode (2) and at least one second sensitivity parameter characterizing the measuring sensitivity of the second measuring electrode (3) are stored,
wherein the analytical unit (23) is designed such that analyzing at least one measuring signal of one of the measuring electrodes (2, 3) allows to determine to which concentration range the analyte concentration belongs, and
the measuring signal of the first measuring electrode (2) is analyzed to determine the analyte concentration by means of the first sensitivity parameter if the analyte concentration belongs to the first concentration range, and
the measuring signal of the second measuring electrode (3) is analyzed to determine the analyte concentration by means of the second sensitivity parameter if the analyte concentration belongs to the second concentration range.

6. Device according to claim 5, **characterized in that** the first concentration range for which the first measuring electrode (2) is optimized, and the second concentration range for which the second measuring electrode (3) is optimized, overlap in an area of overlap, and
the analytical unit (23) is designed such that both the measuring signal of the first measuring electrode (2) and the measuring signal of the second measuring electrode (3) each are analyzed to determine the concentration range or ranges to which the analyte concentration belongs, and analyte concentrations in the area of overlap are determined by means of a statistical analysis from an analytical result of the first measuring electrode (2) and an analytical result of the second measuring electrode (3).

7. Device according to claim 5 or 6, **characterized in that** the first measuring electrode (2) is connected to a first potentiostat (21) and the second measuring electrode (3) is connected to a second potentiostat (22) .

8. Device according to any one of claims 5 to 7, **characterized in that** the measuring voltages applied to the measuring electrodes (2, 3) can be turned on and off separately by means of a control unit (23), whereby the analytical unit (23) is designed such that a local decrease of the analyte concentration, effected by a measuring current for at least one of the measuring electrodes (2, 3) after the measuring voltage is turned on, is recognized and taken into consideration, preferably compensated numerically, in the analysis for determining the analyte concentration in the human or animal body.

## Revendications

1. Système implantable d'électrodes pour un dispositif de mesure d'une concentration en analyte dans un corps humain ou animal, comprenant une première et une seconde électrode de mesure (2, 3) destinées chacune à déterminer des signaux de mesure qui contiennent une information sur la concentration en analyte à mesurer,
dans lequel les électrodes de mesure (2, 3, 7) comprennent à chaque fois une couche d'enzyme (9) avec une enzyme qui produit, par transformation catalytique de l'analyte, des porteurs de charge qui sont détectés pour le signal de mesure,
la première électrode de mesure (2) a une première sensibilité de mesure qui est adaptée à un premier domaine de concentration de l'analyte,
la seconde électrode de mesure (3) a une seconde sensibilité de mesure qui est différente de la première sensibilité de mesure et est adaptée à un second domaine de concentration de l'analyte,
**caractérisé en ce que** les électrodes de mesure (2, 3, 7) ont une contre-électrode (4) commune qui fait partie du système d'électrodes et
**en ce qu'**au moins une des électrodes de mesure (2, 3, 7) comporte une couche de recouvrement (11) qui oppose une résistance à la diffusion de l'analyte, dans lequel une différence entre les sensibilités de mesure de la première électrode de mesure (2) et de la seconde électrode de mesure (3) est obtenue en adaptant la résistance à la diffusion.

2. Système d'électrodes selon la revendication 1, **caractérisé en ce que** les sensibilités de mesure des électrodes de mesure (2, 3) diffèrent d'au moins un facteur 2, de préférence d'au moins un facteur 3, en présence d'une concentration de référence.

3. Système d'électrodes selon l'une des revendications précédentes, **caractérisé en ce que** les sensibilités de mesure différentes de la première et de la seconde électrode de mesure (2, 3) sont obtenues grâce à des couches d'enzyme (9) différentes l'une de l'autre, avec des enzymes différentes et/ou des quantités d'enzyme différentes.

4. Système d'électrodes selon l'une des revendications précédentes, **caractérisé en ce qu'**il comprend au moins une électrode de mesure supplémentaire (7) dont la sensibilité de mesure est adaptée à un autre domaine de concentration de l'analyte.

5. Dispositif de mesure d'une concentration en analyte dans un corps humain ou animal, comprenant :
un système implantable d'électrodes (1) selon l'une des revendications précédentes et
une unité d'analyse reliée au système d'électrodes (1) pour l'analyse de signaux de mesure de la première et de la seconde électrode de mesure (2, 3),
**caractérisé par** une mémoire dans laquelle sont enregistrés au moins un premier paramètre de sensibilité caractérisant la sensibilité de mesure de la première électrode (2) et au moins un second paramètre de sensibilité caractérisant la sensibilité de mesure de la seconde électrode (3),
dans lequel l'unité d'analyse (23) est conçue de façon que l'analyse d'au moins un signal de mesure de l'une des électrodes de mesure (2, 3) permette de déterminer dans quel domaine de concentration se situe la concentration en analyte, et
dans le cas où la concentration en analyte se situe dans le premier domaine de concentration, le signal de mesure de la première électrode de mesure (2) est analysé en utilisant le premier paramètre de sensibilité pour déterminer la concentration en analyte, et
dans le cas où la concentration en analyte se situe dans le second domaine de concentration, le signal de mesure de la seconde électrode de mesure (3) est analysé en utilisant le second paramètre de sensibilité pour déterminer la concentration en analyte.

6. Dispositif selon la revendication 5, **caractérisé en ce que** le premier domaine de concentration, pour lequel la première électrode de mesure (2) est optimisée, et le second domaine de concentration, pour lequel la seconde électrode de mesure (3) est optimisée, se chevauchent dans un domaine de chevauchement, et
l'unité d'analyse (23) est conçue de façon que le signal de mesure de la première électrode de mesure (2), tout comme le signal de mesure de la seconde électrode de mesure (3), soient à chaque fois analysés pour déterminer dans quel ou quels domaine(s) de concentration se situe la concentration en analyte, et les concentrations en analyte se situant dans le domaine de chevauchement sont déterminées grâce à une analyse statistique réalisée à partir d'un résultat d'analyse de la première électrode de mesure (2) et d'un résultat d'analyse de la seconde électrode de mesure (3).

7. Dispositif selon la revendication 5 ou 6, **caractérisé en ce que** la première électrode de mesure (2) est reliée à un premier potentiostat (21) et la seconde électrode de mesure (3) est reliée à un second potentiostat (22).

8. Dispositif selon l'une des revendications 5 à 7, **caractérisé en ce que** les tensions de mesure appliquées respectivement aux électrodes de mesure (2, 3) peuvent être activées et désactivées séparément grâce à une unité de commande (23), dans lequel l'unité d'analyse (23) est conçue de sorte qu'une diminution locale de la concentration en analyte provoquée par un courant de mesure après l'établissement de la tension de mesure pour au moins une des électrodes de mesure (2, 3) soit détectée et soit prise en compte, de préférence compensée numériquement, lors de l'analyse pour déterminer la concentration en analyte dans le corps humain ou animal.
